# EUROPEAN PATENT APPLICATION

(11) **EP 2 130 583 A1**
(43) Date of publication of application: **09.12.2009**
(21) Application number: 08710494.9
(22) Date of filing: 26.02.2008
(51) Int. Cl.: B01J 27/236, C07C 45/29, C07C 47/02, C07C 47/21, C07C 47/232, C07C 47/347, C07C 47/54, C07C 47/542, C07C 47/55, C07C 49/04, C07C 49/12, C07C 49/395, C07C 49/403, C07C 49/413, C07C 49/453, C07C 49/78, C07C 49/792, C07C 201/12, C07C 205/44, C07D 307/48, C07D 317/54

(54) **METHOD FOR PRODUCING CARBONYL COMPOUND**

(30) Priority: 09.03.2007 JP 2007060485
(71) Applicant: Daicel Chemical Industries, Ltd., Kita-ku Osaka-shi Osaka 530-0001 (JP)
(72) Inventor: KANEDA, Kiyotomi, Suita-shi Osaka 565-0871 (JP); YAMASAKI, Noritsugu, Himeji-shi Hyogo 671-1283 (JP)
(74) Representative: Grünecker, Kinkeldey, Stockmair & Schwanhäusser Anwaltssozietät
(86) International application number: PCT/JP2008/000341
(87) International publication number: WO 2008/111282

(57) **Abstract**

Disclosed are a catalyst including a hydrotalcite and, immobilized on a surface thereof, particles of at least one metal selected from the group consisting of Cu, Ag, and Au; a method for producing a carbonyl compound through dehydrogenation of an alcohol in the presence of the catalyst; and a method for producing a carbonyl compound through dehydrogenation of an alcohol in the presence of a catalyst including a hydrotalcite and, immobilized on a surface thereof, particles of a metal, in which dehydrogenation is performed in the absence of oxygen.

## Description

### Technical Field

The present invention relates to methods for producing carbonyl compounds through dehydrogenation of alcohols to give corresponding carbonyl compounds.

### Background Art

Conversion of alcohols to carbonyl compounds is one of most important reactions in organic synthesis, and a variety of catalysts and reaction systems using the catalysts have been examined. Typically, Japanese Unexamined Patent Application Publication (JP-A) No. 2000-86245 (Patent Document 1) discloses production of a corresponding ketone or carboxylic acid through oxidation of an alcohol catalyzed by a synthetic hydrotalcite containing ruthenium (Ru) in its backbone. Japanese Unexamined Patent Application Publication (JP-A) No. 2002-274852 (Patent Document 2) discloses a metal-immobilized hydrotalcite including a synthetic hydrotalcite containing manganese (Mn) in its backbone; and Ru immobilized on a surface of the synthetic hydrotalcite. The document also discloses use of the metal-immobilized hydrotalcite as a catalyst in a reaction for oxidizing an alcohol to a corresponding carbonyl compound. These techniques, however, are disadvantageous typically in that preparation of the synthetic hydrotalcite requires complicated procedures and that raw-material alcohols are restricted in their structure. It is therefore desirable to provide a reaction system which enables efficient oxidation (dehydrogenation) especially of cyclohexanol and other alicyclic alcohols having low reactivity.

Additionally, it is also desirable to provide a method for producing a carbonyl compound, in which an oxidation reaction is performed without using a hydrogen (H₂) acceptor such as molecular oxygen. This is because such method is environmentally friendly, and its aftertreatment can be simply carried out.

Patent Document 1: JP-A No. 2000-86245
Patent Document 2: JP-A No. 2002-274852

### Disclosure of Invention

### Problems to be Solved by the Invention

Accordingly, an object of the present invention is to provide a catalyst which is usable in a method for producing a corresponding carbonyl compound through dehydrogenation of an alcohol, which enables simple and efficient production of the carbonyl compound, and which can be applied to a wide variety of alcohols to produce a variety of carbonyl compounds; and to provide a method for producing a carbonyl compound using the catalyst.

Another object of the present invention is to provide a method for producing a corresponding carbonyl compound through dehydrogenation of an alcohol, in which dehydrogenation can be performed without using a hydrogen acceptor such as molecular oxygen.

### Means for Solving the Problems

After intensive investigations to achieve the objects, the present inventors have found that use of a catalyst including a hydrotalcite and, immobilized on a surface thereof, particles of a specific metal enables efficient production of carbonyl compounds from a wide variety of alcohols; and that use of a catalyst including a hydrotalcite and, immobilized on a surface thereof, particles of a metal enables efficient production of carbonyl compounds without using a hydrogen (H₂) acceptor. The present invention has been made based on these findings.

Specifically, in an embodiment, the present invention provides a catalyst which includes a hydrotalcite and, immobilized on a surface thereof, particles of at least one metal selected from the group consisting of copper (Cu), silver (Ag), and gold (Au).

In another embodiment, the present invention provides a method for producing a carbonyl compound, which method includes the step of dehydrogenating an alcohol in the presence of a catalyst including a hydrotalcite and, immobilized on a surface thereof, particles of at least one metal selected from the group consisting of Cu, Ag, and Au.

In yet another embodiment, the present invention provides a method for producing a carbonyl compound, which method includes the step of dehydrogenating an alcohol in the presence of a catalyst including a hydrotalcite and, immobilized on a surface thereof, particles of a metal, in which dehydrogenation is carried out in the absence of oxygen.

### Advantages

The present invention enables efficient dehydrogenation of alcohols by a simple operation to give corresponding carbonyl compounds in high yields. The methods according to the present invention are applicable to a wide variety of alcohols and, above all, enable efficient dehydrogenation of alicyclic alcohols by the catalysis of a solid catalyst in a liquid phase under mild conditions, in contrast to known techniques.

Dehydrogenation, if conducted in the absence of oxygen by the method according to the present invention, does not cause by-products other than hydrogen, and this enables isolation of a target compound by a simple operation.

The catalyst according to the present invention, which includes a hydrotalcite and, immobilized on a surface thereof, metal particles, can be prepared by a simple operation, is easily recoverable and reusable after the completion of reaction, and is extremely advantageous from the viewpoint of "green chemistry (sustainable chemistry)". Best Modes for Carrying Out the Invention

### [Catalyst Including Metal Particles Immobilized On Hydrotalcite Surface]

A catalyst according to the present invention including metal particles immobilized on a hydrotalcite surface is a solid catalyst that includes a hydrotalcite, and immobilized on its surface, particles of a metal. The hydrotalcite is not especially limited and can be a naturally-occurring hydrotalcite, a synthetic hydrotalcite, or a synthetic hydrotalcite-like compound. Exemplary hydrotalcites for use herein include naturally-occurring or synthetic hydrotalcites represented by following General Formula (1):

M^{II}₈₋ₓM^{III}ₓ(OH)₁₆A·nH₂O (1)

wherein M^{II} is at least one bivalent metal selected from Mg²⁺, Fe²⁺, Zn²⁺, Ca²⁺, Li²⁺, Ni²⁺, Co²⁺, Cu², and Mn²⁺; M^{III} is at least one trivalent metal selected from Al³⁺, Fe³⁺, Mn³⁺, and Ru³⁺; "x" denotes an integer of from 1 to 7; "A" represents a bivalent anion; and "n" denotes a number of from 0 to 30, or represented by following General Formula (2):

[Mg²⁺_{1-y}Al³⁺_{y}(OH)₂]_{y/s}·mH₂O]^{y-} (2)

wherein "y" denotes a number satisfying the condition: 0.20≤y≤0.33; D^{s-} represents an anion having a valency "s"; and "m" denotes an integer of from 0 to 30.
Preferred hydrotalcites are those of General Formula (1) in which M^{II} is Mg²⁺, M^{III} is Al³⁺, and "A" is CO₃²⁻. An exemplary hydrotalcite advantageously usable herein is a hydrotalcite represented by Mg₆Al₂(OH)₁₆CO₃.

Exemplary metal species immobilized on a hydrotalcite surface include transition metal elements. Preferred transition metal elements include Group 8 elements (e.g., Fe, Ru, and Os), Group 9 elements (e.g., Co and Ir), Group 10 elements (e.g., Ni, Pd, and Pt), and Group 11 elements (e.g., Cu, Ag, and Au). Among them, Cu, Ag, and Au are advantageously usable. Each of different metals can be selected and used alone or in combination.

A process for immobilizing particles of a metal to a surface of a hydrotalcite (metal immobilization process) is not especially limited. For example, immobilization can be performed by mixing and stirring a solution of a compound containing the metal with the hydrotalcite to immobilize metal ions to the surface of the hydrotalcite; and subsequently reducing the metal ions through a suitable procedure. Exemplary metal-containing compounds usable herein include metal salts such as chlorides, bromides, iodides, nitrates, sulfates, and phosphates; as well as metal complexes and other compounds. The solvent is not especially limited, as long as the metallic compound to be used is soluble therein, and examples thereof include water, acetone, and alcohols. Though not especially limited, the concentration of the metal-containing compound in the solution in the metal immobilization process can be selected within a range of, for example, from 0.1 to 1000 mM. The stirring temperature is, for example, from about 20°C to about 150°C, preferably from about 50°C to about 110°C, and especially preferably from about 60°C to about 80°C. Though not especially limited, the metal content of the catalyst including metal particles immobilized on a hydrotalcite surface can be selected within a range of, for example, from 0.01 to 10 mmol, and preferably from 2.5 to 10 mmol, per 1 g of the hydrotalcite. Tough varying depending on the stirring temperature, the stirring time (duration) can be selected within a range of, for example, from 1 to 72 hours, and preferably from 10 to 24 hours. A product after the completion of stirring may be washed typically with water or an organic solvent and dried typically by vacuum drying.
The reduction can be performed through a treatment with a reducing agent such as hydrogen, hydrazine, formaldehyde, potassium borohydride, or sodium borohydride. Typically, when a reduction treatment is carried out with hydrogen, the reduction can be performed by firing a hydrotalcite bearing metal ions immobilized thereon at 20°C to 110°C for about 10 to about 24 hours to convert the metal to an oxide, and carrying out stirring at 150°C to 180°C in a hydrogen atmosphere or in a vacuum for about 0.5 hour.

### [Production of Carbonyl Compounds]

The catalyst according to the present invention including metal particles immobilized on a hydrotalcite surface is usable for the dehydrogenation of various alcohols to give corresponding carbonyl compounds. The method according to the present invention enables efficient dehydrogenation of both alicyclic alcohols and acyclic alcohols (chain aliphatic alcohols) to give corresponding carbonyl compounds in high yields.

Alicyclic alcohols are compounds each having an alicyclic hydrocarbon ring whose constitutive carbon atom having a hydroxyl group bound thereto. Exemplary alicyclic alcohols include saturated alicyclic alcohols such as cyclobutanol, cyclopentanol, cyclohexanol, cycloheptanol, cyclooctanol, and cyclododecanol; unsaturated alicyclic alcohols such as cyclopentenol and cyclohexenol; and saturated or unsaturated polycyclic alicyclic alcohols such as 1-adamantanol and 2-adamantanol.

Examples of acyclic (noncyclic) alcohols (chain aliphatic alcohols) include saturated aliphatic linear primary alcohols such as methanol, ethanol, 1-propanol, 1-butanol, 1-pentanol, 1-hexanol, 1-heptanol, 1-octanol, 1-nonanol, and 1-decanol; saturated aliphatic linear secondary alcohols such as 2-propanol, 2-butanol, 2-pentanol, 3-pentanol, 2-hexanol, 3-hexanol, 2-heptanol, 3-heptanol, 2-octanol, 3-octanol, 4-octanol, 2-nonanol, and 2-decanol; unsaturated linear primary alcohols such as allyl alcohol, 3-buten-1-ol, 2-hexen-1-ol, and 2,4-hexadien-1-ol; unsaturated linear secondary alcohols such as 4-penten-2-ol; saturated aliphatic branched-chain primary alcohols such as 3-methyl-1-butanol; saturated aliphatic branched-chain secondary alcohols such as 3-methyl-2-butanol; unsaturated aliphatic branched-chain primary alcohols such as 3-methyl-2-buten-1-ol and 4-methyl-2-propen-1-ol; unsaturated aliphatic branched-chain secondary alcohols such as 5-methyl-3-hexen-2-ol; and aliphatic chain alcohols having a saturated or unsaturated alicyclic group as a substituent, such as 1-hydroxymethyladamantane, 1-cyclohexyl-2-propanol, myrtenol, and perillyl alcohol.

The alicyclic alcohols and acyclic alcohols (chain aliphatic alcohols) may each have an aromatic hydrocarbon group and/or a heterocyclic group as a substituent. The aromatic hydrocarbon group may be one containing a single ring, such as phenyl group; or a one containing fused two or more rings, such as naphthyl group or azulenyl group. The aromatic hydrocarbon group may have one or more substituents selected typically from nitro groups, sulfo groups, chloro groups, fluoro groups, alkyl groups, alkenyl groups, and alkynyl groups. Exemplary alcohols having an aromatic hydrocarbon group as a substituent include benzyl alcohol, p-methylbenzyl alcohol, p-isopropylbenzyl alcohol, p-nitrobenzyl alcohol, p-chlorobenzyl alcohol, 1-phenylethanol, benzhydrol, cinnamyl alcohol, and phenylcyclopropylmethanol.

Examples of the heterocyclic group include oxygen-containing heterocyclic groups such as furanyl group and benzodioxole group; nitrogen-containing heterocyclic groups such as pyrrolyl group; and sulfur-containing heterocyclic groups such as thionyl group.

Exemplary alcohols to be dehydrogenated by the method according to the present invention further include hydroxyketones and hydroxyaldehydes. Exemplary hydroxyketones include α-hydroxyketones such as 6-hydroxydecan-5-one, acetoin, and benzoin. Dehydrogenation of such α-hydroxyketones gives corresponding α-diketones.

Dehydrogenation of the alcohols in the presence of the catalyst including metal particles immobilized on a hydrotalcite surface gives corresponding carbonyl compounds. Typically, dehydrogenation of a primary alcohol, if used as a raw material, gives a corresponding aldehyde; and dehydrogenation of a raw-material secondary alcohol gives a corresponding ketone.

The reaction can be performed in a liquid phase or in a gaseous phase. The reaction herein is preferably performed in a liquid phase in view typically of workability.

The reaction can be performed in the presence of, or in the absence of, a solvent. The solvent is not especially limited, as long as a raw-material alcohol can be dissolved therein and a reaction is not adversely affected, and can be chosen from among known or common solvents. Exemplary solvents include aromatic hydrocarbons, such as benzene, toluene, xylene, chlorobenzene, and nitrobenzene; aliphatic hydrocarbons such as pentane, hexane, heptane, octane, cyclohexane, and methylcyclohexane; ethers such as diethyl ether, tetrahydrofuran, and tetrahydropyran; nitriles such as acetonitrile and benzonitrile; amides such as acetamide, dimethylacetamide, dimethylformamide, diethylformamide, and N-methylpyrrolidone; esters such as ethyl acetate, propyl acetate, and butyl acetate; water; and mixtures of these solvents. Among them, aromatic hydrocarbons and aliphatic hydrocarbons are preferably used, of which toluene and methylcyclohexane are more preferably used.

The reaction may be performed in an atmosphere of an inert gas such as nitrogen or argon gas; or can be performed in the air or in an oxygen atmosphere. A hydrogen (H₂) acceptor (oxidizing agent) such as oxygen is used according to known techniques for the production of carbonyl compounds through oxidation of alcohols. However, the dehydrogenation of alcohols according to the present invention can be performed even without using a hydrogen (H₂) acceptor such as molecular oxygen. Surprisingly, a catalyst including particles of a metal of some type immobilized on a hydrotalcite surface, if used in hydrogenation of an alcohol in the absence of oxygen, may exhibit significantly improved catalytic activity to give a corresponding carbonyl compound in a dramatically high yield as compared to the dehydrogenation in an oxygen atmosphere. Typically, a catalyst including copper (Cu) particles immobilized on a hydrotalcite surface shows dramatically increased activity in the absence of oxygen. This tendency is remarkable especially in dehydrogenation of secondary alcohols. Among such secondary alcohols, alicyclic alcohols, such as cyclohexanol, cyclooctanol, and adamantanol, show typically low reactivity. The method according to the present invention enables efficient dehydrogenation of such alicyclic alcohols to give corresponding cyclic ketones in high yields. The phrase "the absence of oxygen" refers to the case where the oxygen concentration of a gaseous phase in the reaction system is 1% or less, preferably 0.1% or less, and especially preferably 0.01% or less. It is enough that the inside of the reaction system is sufficiently purged by an inert gas such as nitrogen or argon gas before the initiation of the reaction.

A reaction, if performed in the absence of a hydrogen (H₂) acceptor, H₂ formed as a by-product can be easily removed, and this facilitates isolation and purification of the target product.

As has been described above, Au, Ag, and Cu are especially preferred as metal species to be immobilized on a hydrotalcite surface. A catalyst system, if using at least one of these metals, is extremely highly applicable to substrates and shows a high activity on dehydrogenation of not only the secondary alcohols including alicyclic alcohols, but also primary alcohols. In addition, the catalyst system allows dehydrogenation to proceed efficiently to give corresponding carbonyl compounds, even when the alcohols have unsaturated bonds and/or substituents such as aromatic hydrocarbon groups, heterocyclic groups, carbonyl groups, aldehyde groups, and carboxyl groups.

The amount of the catalyst can be suitably set according typically to the type of a raw-material alcohol and the type of a metal immobilized on a hydrotalcite surface. Typically, the amount in terms of the metal immobilized on a hydrotalcite surface can be chosen within ranges of, for example, from 0.0001 to 20 percent by mole, preferably from 0.0005 to 10 percent by mole, and especially preferably from 2 to 8 percent by mole, relative to the raw-material alcohol.

The reaction can be performed under normal atmospheric pressure or under a pressure (under a load), but it is preferably performed under normal atmospheric pressure in consideration typically of workability. Though not especially limited and can be set according to the type of a raw-material alcohol and the type of a metal immobilized on a hydrotalcite surface, the reaction temperature can be chosen within ranges of, for example, from 0°C to 200°C, preferably from 60°C to 180°C, and especially preferably from 100°C to 160°C.

Though not especially limited and can be suitably set according typically to the types of the raw-material alcohol and solvent and to the reaction temperature, the reaction time (duration) can be chosen within ranges of, for example, from 0.5 to 200 hours, and preferably from 1 to 150 hours. The reaction can be performed according to a common system such as a batch system, a semi-batch system, or a continuous system. Reaction products after the completion of the reaction can be separated and purified through a separation procedure such as filtration, concentration, distillation, extraction, crystallization, recrystallization, adsorption, or column chromatography, or any combination of them.

After the completion of the reaction, the catalyst including metal particles immobilized on a hydrotalcite surface is recovered through an operation such as filtration or centrifugal separation, and can be reused as a dehydrogenate catalyst for alcohols after no further treatment or after washing typically with water or an organic solvent according to necessity. The catalyst including metal particles immobilized on a hydrotalcite surface, even when reused in reactions of alcohols, does not suffer from decrease in its catalytic activity and can give corresponding carbonyl compounds in high yields.

### EXAMPLES

The present invention will be illustrated in further detail with reference to several examples below. It should be noted, however, these examples are never construed to limit the scope of the present invention.

### (EXAMPLE 1-0): Preparation of Catalyst Including Au Particles Immobilized on Hydrotalcite Surface (Au/HT)

To a solution of 0.124 g of chloroauric acid (HAuCl₄·xH₂O) in 50 mol of ion exchanged water, was added 1.0 g of a hydrotalcite [Mg₆Al₂(OH)₁₆CO₃], followed by addition of a 25% aqueous ammonia solution and stirring at room temperature for 2 hours. The resulting solids were collected by filtration under reduced pressure, washed with deionized water, dried under reduced pressure, further dried at room temperature in a vacuum, treated at 150°C in a vacuum for 0.5 hour, and thereby yielded a catalyst including Au particles immobilized on a hydrotalcite surface (Au/HT).

### (EXAMPLE 1-1)

A mixture of 1 mmol of cyclooctanol, 5 mL of toluene, and 0.2 g (Au: 6.0 percent by mole (Au content: 6 percent by mole per 1 mole of the substrate; hereinafter the same)) of the catalyst prepared from Example 1-0 and including Au particles immobilized on a hydrotalcite surface was stirred at 110°C in an argon (Ar) atmosphere for 3 hours and thereby yielded a corresponding carbonyl compound (cyclooctanone) in a yield equivalent to a gas chromatographic (GC) yield of 94%.

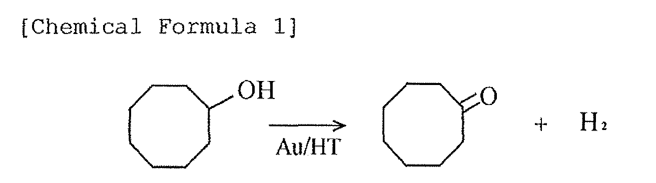

### (EXAMPLE 1-2)

A mixture of 1 mmol of cyclooctanol, 5 mL of water, and 0.2 g (Au: 6.0 percent by mole) of the catalyst prepared from Example 1-0 and including Au particles immobilized on a hydrotalcite surface was stirred at 110°C in an argon (Ar) atmosphere for 20 hours and thereby yielded a corresponding carbonyl compound (cyclooctanone) in a yield equivalent to a gas chromatographic (GC) yield of 99% or more.

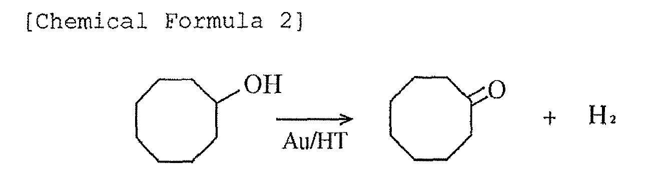

### (EXAMPLE 1-3)

A mixture of 1 mmol of 1-phenylethanol, 5 mL of toluene, and 0.1 g (Au: 3.0 percent by mole) of the catalyst prepared from Example 1-0 and including Au particles immobilized on a hydrotalcite surface was stirred at 110°C in an argon atmosphere for 9 hours and thereby yielded a corresponding carbonyl compound (acetophenone) in a yield equivalent to a gas chromatographic (GC) yield of 99%.

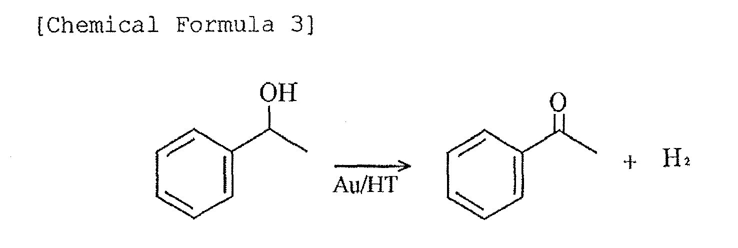

### (EXAMPLE 1-4)

A mixture of 1 mmol of benzyl alcohol, 5 mL of toluene, and 0.1 g (Au: 3.0 percent by mole) of the catalyst prepared from Example 1-0 and including Au particles immobilized on a hydrotalcite surface was stirred at 110°C in an argon atmosphere for 21 hours and thereby yielded a corresponding carbonyl compound (benzaldehyde) in a yield equivalent to a gas chromatographic (GC) yield of 81%.

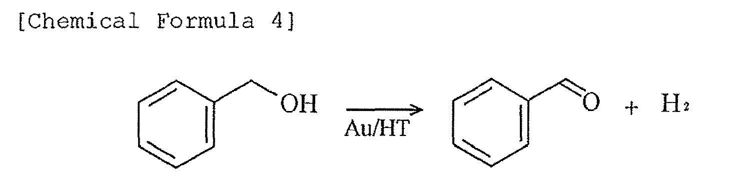

### (EXAMPLE 1-5)

A mixture of 0.5 mL of 1-phenylethanol and 0.1 g (Au: 3.0 percent by mole) of the catalyst prepared from Example 1-0 and including Au particles immobilized on a hydrotalcite surface was stirred at 150°C in an argon atmosphere for 140 hours and thereby yielded a corresponding carbonyl compound (acetophenone) in a yield equivalent to a gas chromatographic (GC) yield of 97%, with a turnover number (TON) of 2500.

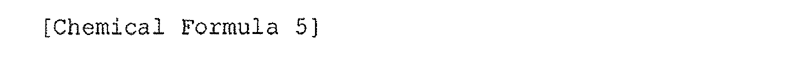

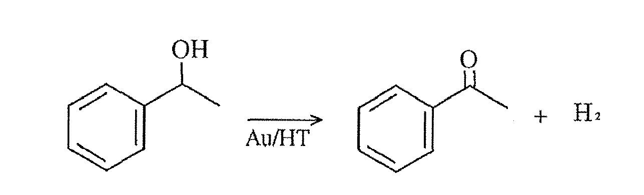

### (EXAMPLE 1-6)

A mixture of 0.5 mol of cyclohexanol and 0.1 g (Au: 3.0 percent by mole) of the catalyst prepared from Example 1-0 and including Au particles immobilized on a hydrotalcite surface was stirred at 150°C in an argon atmosphere for 98 hours and thereby yielded a corresponding carbonyl compound (cyclohexanone) in a yield equivalent to a gas chromatographic (GC) yield of 89%, with a turnover number (TON) of 1480.

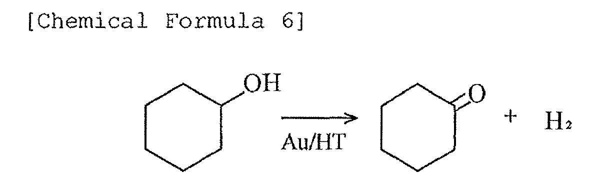

### (EXAMPLE 2-0): Preparation of Catalyst Including Ag Particles Immobilized on Hydrotalcite Surface (Ag/HT)

To a solution of 0.085 g of silver nitrate in 4 mol of ion exchanged water, was added a 25% aqueous ammonia solution to adjust pH to 6.8, followed by addition of 11.0 g of a hydrotalcite [Mg₆Al₂(OH)₁₆CO₃] and stirring at room temperature for 20 minutes. Next, 35 mL of ion exchanged water was added, the resulting solids were collected by filtration under reduced pressure, washed with deionized water, dried under reduced pressure, fired at 110°C overnight, reduced at 180°C in a hydrogen atmosphere for 0.5 hour, and thereby yielded a catalyst including Ag particles immobilized on a hydrotalcite surface (Ag/HT).

### (EXAMPLE 2-1)

A mixture of 1 mmol of cyclopentanol, 5 mL of toluene, and 0.1 g of the catalyst prepared from Example 2-0 and including Ag particles immobilized on a hydrotalcite surface was stirred at 110°C in an argon atmosphere for 24 hours and thereby yielded a corresponding carbonyl compound (cyclopentanone) in a yield equivalent to a gas chromatographic (GC) yield of 70%, with a selectivity for chclopentanone of 99% or more.

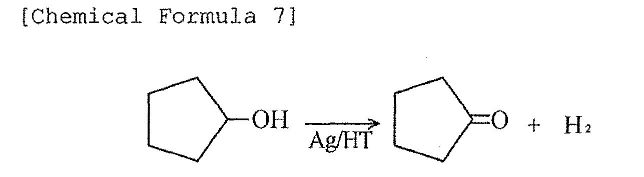

### (EXAMPLE 2-2)

A mixture of 1 mmol of cyclooctanol, 5 mL of toluene, and 0.1 g of the catalyst prepared from Example 2-0 and including Ag particles immobilized on a hydrotalcite surface was stirred at 110°C in an argon atmosphere for 22 hours and thereby yielded a corresponding carbonyl compound (cyclooctanone) in a yield equivalent to a gas chromatographic (GC) yield of 99%, with a selectivity for cyclooctanone of 99% or more.

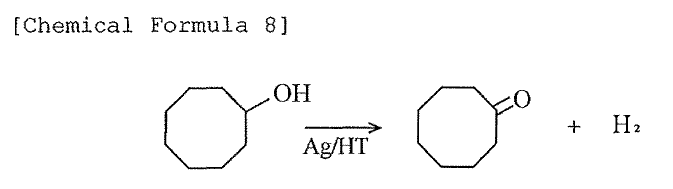

### (EXAMPLE 2-3)

A mixture of 1 mmol of cyclododecanol, 5 mL of toluene, and 0.1 g of the catalyst prepared from Example 2-0 and including Ag particles immobilized on a hydrotalcite surface was stirred at 110°C in an argon atmosphere for 21 hours and thereby yielded a corresponding carbonyl compound (cyclododecanol) in a yield equivalent to a gas chromatographic (GC) yield of 98%, with a selectivity for cyclododecanone of 99% or more.

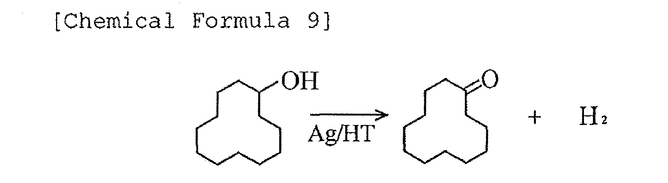

### (EXAMPLE 2-4)

A mixture of 1 mmol of benzyl alcohol, 5 mL of toluene, and 0.1 g of the catalyst prepared from Example 2-0 and including Ag particles immobilized on a hydrotalcite surface was stirred at 110°C in an argon atmosphere for 24 hours and thereby yielded a corresponding carbonyl compound (benzaldehyde) in a yield equivalent to a gas chromatographic (GC) yield of 98%, with a selectivity for benzaldehyde of 99% or more.

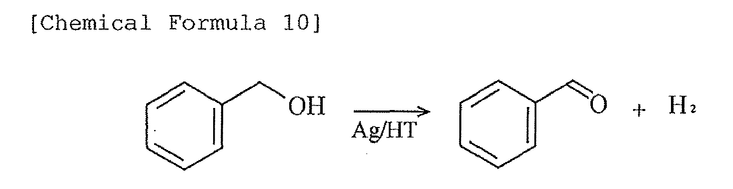

### (EXAMPLE 2-5)

A mixture of 1 mmol of p-methylbenzyl alcohol, 5 mL of toluene, and 0.1 g of the catalyst prepared from Example 2-0 and including Ag particles immobilized on a hydrotalcite surface was stirred at 110°C in an argon atmosphere for 24 hours and thereby yielded a corresponding carbonyl compound (p-methylbenzaldehyde) in a yield equivalent to a gas chromatographic (GC) yield of 95%, with a selectivity for p-methylbenzaldehyde of 99% or more.

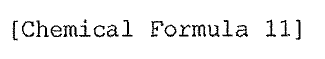

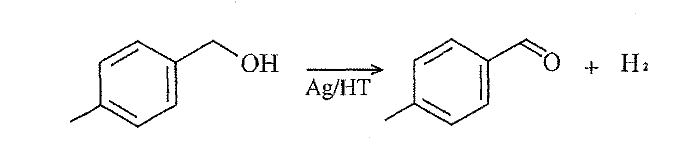

### (EXAMPLE 2-6)

A mixture of 1 mmol of p-isopropylbenzyl alcohol, 5 mL of toluene, and 0.1 g of the catalyst prepared from Example 2-0 and including Ag particles immobilized on a hydrotalcite surface was stirred at 110°C in an argon atmosphere for 24 hours and thereby yielded a corresponding carbonyl compound (p-isopropylbenzaldehyde) in a yield equivalent to a gas chromatographic (GC) yield of 82%, with a selectivity for p-isopropylbenzaldehyde of 99% or more.

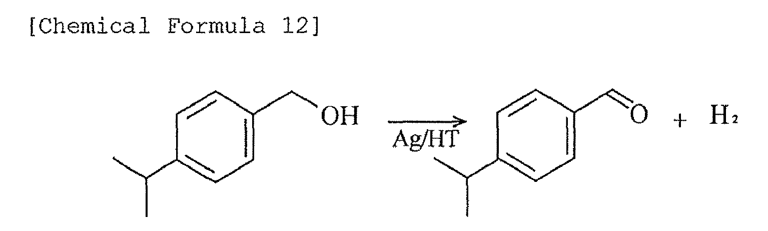

### (EXAMPLE 2-7)

A mixture of 1 mmol of p-nitrobenzyl alcohol, 5 mL of toluene, and 0.1 g of the catalyst prepared from Example 2-0 and including Ag particles immobilized on a hydrotalcite surface was stirred at 110°C in an argon atmosphere for 48 hours and thereby yielded a corresponding carbonyl compound (p-nitrobenzaldehyde) in a yield equivalent to a gas chromatographic (GC) yield of 76%, with a selectivity for p-nitrobenzaldehyde of 98%.

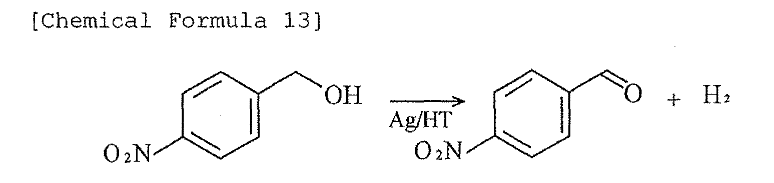

### (EXAMPLE 2-8)

A mixture of 1 mmol of p-chlorobenzyl alcohol, 5 mL of toluene, and 0.1 g of the catalyst prepared from Example 2-0 and including Ag particles immobilized on a hydrotalcite surface was stirred at 110°C in an argon atmosphere for 120 hours and thereby yielded a corresponding carbonyl compound (p-chlorobenzaldehyde) in a yield equivalent to a gas chromatographic (GC) yield of 73%, with a selectivity for p-chlorobenzaldehyde of 99% or more.

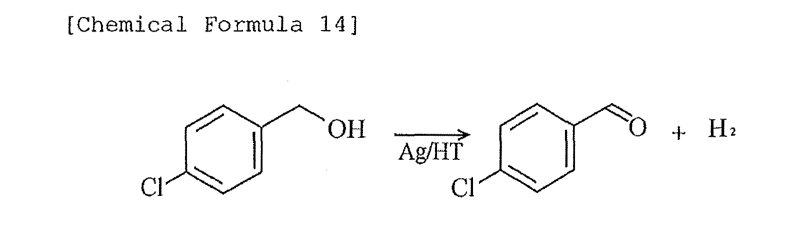

### (EXAMPLE 2-9)

A mixture of 1 mmol of 1,3-benzodioxole-5-methanol (piperonyl alcohol), 5 mL of toluene, and 0.1 g of the catalyst prepared from Example 2-0 and including Ag particles immobilized on a hydrotalcite surface was stirred at 110°C in an argon atmosphere for 45 hours and thereby yielded a corresponding carbonyl compound (1,3-benzodioxole-5-carbaldehyde) in a yield equivalent to a gas chromatographic (GC) yield of 93%, with a selectivity for 1,3-benzodioxole-5-carnaldehyde of 99% or more.

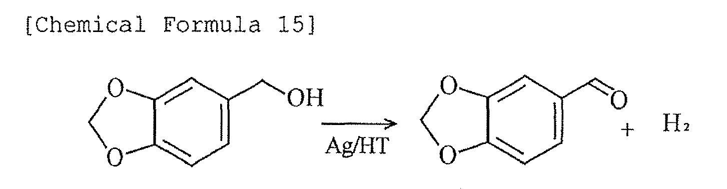

### (EXAMPLE 2-10)

A mixture of 1 mmol of 3-phenyl-2-propen-1-ol (cinnamyl alcohol), 5 mL of toluene, and 0.1 g of the catalyst prepared from Example 2-0 and including Ag particles immobilized on a hydrotalcite surface was stirred at 110°C in an argon atmosphere for 94 hours and thereby yielded a corresponding carbonyl compound (cinnamaldehyde) in a yield equivalent to a gas chromatographic (GC) yield of 84%, with a selectivity for cinnamaldehyde of 99% or more.

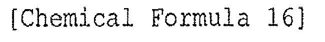

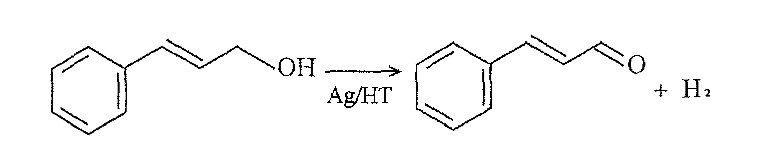

### (EXAMPLE 2-11)

A mixture of 0.5 mmol of 3-methyl-2-buten-1-ol and 0.01 g of the catalyst prepared from Example 2-0 and including Ag particles immobilized on a hydrotalcite surface was stirred at 110°C in an argon atmosphere for 45 hours and thereby yielded a corresponding carbonyl compound (3-methyl-2-buten-1-one) in a yield equivalent to a gas chromatographic (GC) yield of 59%, with a selectivity for 3-methyl-2-buten-1-one of 99% or more.

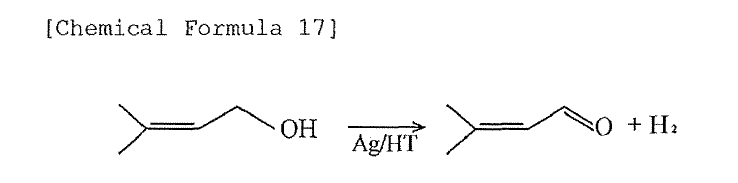

### (EXAMPLE 2-12)

A mixture of 1 mmol of 2,4-hexadien-1-ol, 5 mL of toluene, and 0.1 g of the catalyst prepared from Example 2-0 and including Ag particles immobilized on a hydrotalcite surface was stirred at 110°C in an argon atmosphere for 96 hours and thereby yielded a corresponding carbonyl compound (2,4-hexadien-1-one) in a yield equivalent to a gas chromatographic (GC) yield of 55%, with a selectivity for 2,4-hexadien-1-one of 99% or more.

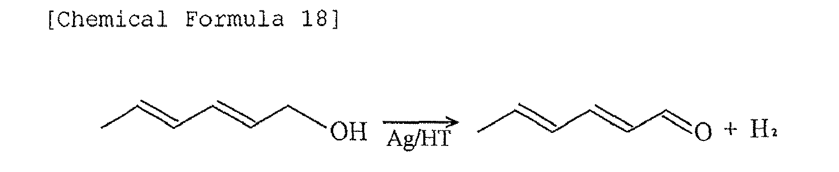

### (EXAMPLE 2-13)

A mixture of 1 mmol of 1-phenylethanol, 5 mL of toluene, and 0.1 g of the catalyst prepared from Example 2-0 and including Ag particles immobilized on a hydrotalcite surface was stirred at 110°C in an argon atmosphere for 20 hours and thereby yielded a corresponding carbonyl compound (acetophenone) in a yield equivalent to a gas chromatographic (GC) yield of 99%, with a selectivity for acetophenone of 99% or more.

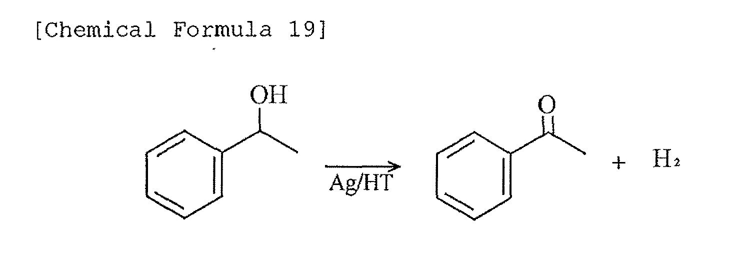

### (EXAMPLE 2-14)

A mixture of 5 mmol of 1-phenylethanol and 0.01 g of the catalyst prepared from Example 2-0 and including Ag particles immobilized on a hydrotalcite surface was stirred at 100°C in an argon atmosphere for 72 hours and thereby yielded a corresponding carbonyl compound (acetophenone) in a yield equivalent to a gas chromatographic (GC) yield of 99%, with a selectivity for acetophenone of 99% or more.

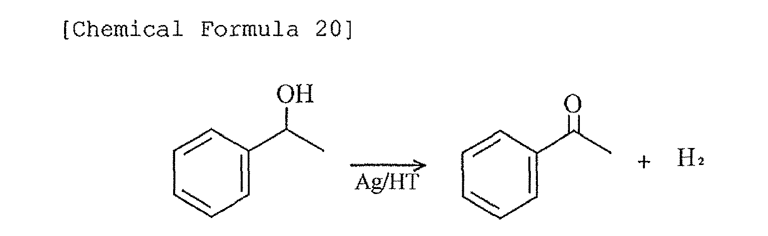

### (EXAMPLE 2-15)

A mixture of 1 mmol of 2-octanol, 5 mL of toluene, and 0.1 g of the catalyst prepared from Example 2-0 and including Ag particles immobilized on a hydrotalcite surface was stirred at 110°C in an argon atmosphere for 26 hours and thereby yielded a corresponding carbonyl compound (2-octanone) in a yield equivalent to a gas chromatographic (GC) yield of 99%, with a selectivity for 2-octanone of 99% or more.

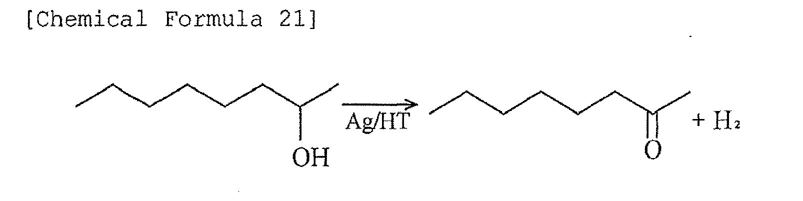

### (EXAMPLE 2-16)

A mixture of 1 mmol of 2-adamantanol, 5 mL of toluene, and 0.1 g of the catalyst prepared from Example 2-0 and including Ag particles immobilized on a hydrotalcite surface was stirred at 110°C in an argon atmosphere for 20 hours and thereby yielded a corresponding carbonyl compound (2-adamantanone) in a yield equivalent to a gas chromatographic (GC) yield of 97%, with a selectivity for 2-adamantanone of 99% or more.

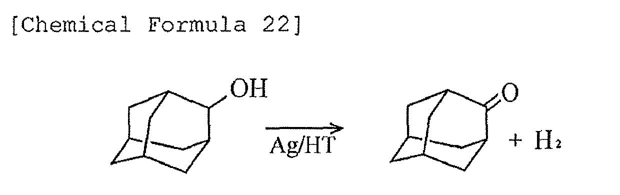

### (EXAMPLE 2-17)

A mixture of 1 mmol of 6-hydroxydecan-5-one, 5 mL of toluene, and 0.1 g of the catalyst prepared from Example 2-0 and including Ag particles immobilized on a hydrotalcite surface was stirred at 110°C in an argon atmosphere for 48 hours and thereby yielded a corresponding carbonyl compound (5,6-decanedione) in a yield equivalent to a gas chromatographic (GC) yield of 98%, with a selectivity for 5,6-decanedione of 99% or more.

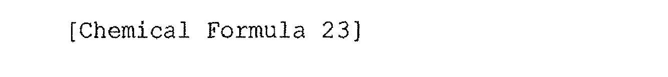

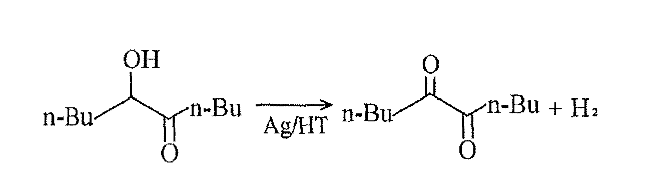

### (EXAMPLE 2-18)

A mixture of 1 mol of 1-octanol, 5 mL of toluene, and 0.1 g of the catalyst prepared from Example 2-0 and including Ag particles immobilized on a hydrotalcite surface was stirred at 110°C in an argon atmosphere for 24 hours and thereby yielded a corresponding carbonyl compound (1-octanal) in a yield equivalent to a gas chromatographic (GC) yield of 27%, with a selectivity for 1-octanal of 99% or more.

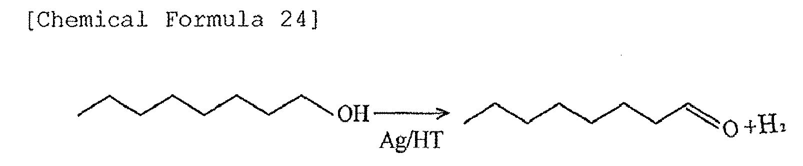

### (EXAMPLE 2-19)

A mixture of 1 mmol of 2-furanmethanol, 5 mL of toluene, and 0.1 g of the catalyst prepared from Example 2-0 and including Ag particles immobilized on a hydrotalcite surface was stirred at 110°C in an argon atmosphere for 120 hours and thereby yielded a corresponding carbonyl compound (2-furaldehyde) in a yield equivalent to a gas chromatographic (GC) yield of 70%, with a selectivity for 2-furaldehyde of 99% or more.

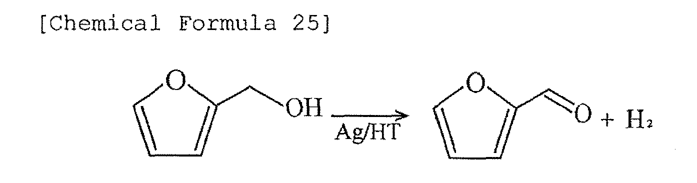

### (EXAMPLE 2-20)

The procedure of Example 2-2 was performed, except for using methylcyclohexane instead of toluene, and thereby yielded a corresponding carbonyl compound (cyclooctanone) in a yield equivalent to a gas chromatographic (GC) yield of 65%.

### (EXAMPLE 2-21)

### [Reuse of Ag/HT]

After the completion of the reaction in Example 2-2, the Ag/HT catalyst was recovered by centrifugal separation and, without further treatment, reused as a catalyst for dehydrogenation of cyclooctanol by the procedure of Example 2-2. The Ag/HT catalyst was reused a total of 10 times, and the yields of resulting cyclooctanone in ten operations in terms of gas chromatographic (GC) yield were determined. Table 1 shows the yields of cyclooctanone. The catalyst including Ag particles immobilized on a hydrotalcite surface showed substantially no decrease in activity even after reuses of ten times.

[Table 1]

**TABLE 1**

| | Yield (%) | Table 1 | Yield (%) |
|---|---|---|---|
| Example 2-2 | 99 | Sixth reuse | 82 |
| First reuse | 99 | Seventh reuse | 96 |
| Second reuse | 96 | Eighth reuse | 99 |
| Third reuse | 99 | Ninth reuse | 97 |
| Forth reuse | 99 | Tenth reuse | 94 |
| Fifth reuse | 98 | | |

### (COMPARATIVE EXAMPLE 1)

The procedure of Example 2-2 was performed, except for using Ag immobilized on silicon dioxide (Ag/SiO₂) instead of Ag/HT, and thereby yielded a corresponding carbonyl compound (cyclooctanone) in a yield equivalent to a gas chromatographic (GC) yield of 5%.

### (COMPARATIVE EXAMPLE 2)

The procedure of Example 2-2 was performed, except for using Ag immobilized on titanium dioxide (Ag/TiO₂) instead of Ag/HT, and thereby yielded a corresponding carbonyl compound (cyclooctanone) in a yield equivalent to a gas chromatographic (GC) yield of 5%.

### (COMPARATIVE EXAMPLE 3)

The procedure of Example 2-2 was performed, except for using Ag₂O instead of Ag/HT, and thereby yielded a corresponding carbonyl compound (cyclooctanone) in a yield equivalent to a gas chromatographic (GC) yield of 23%.

### (COMPARATIVE EXAMPLE 4)

The procedure of Example 2-2 was performed, except for using AgNO₃ instead of Ag/HT, and thereby yielded a corresponding carbonyl compound (cyclooctanone) in a yield equivalent to a gas chromatographic (GC) yield of 7%.

### (EXAMPLE 3-0)

### Preparation of Catalyst Including Cu Particles Immobilized on Hydrotalcite Surface (Cu/HT)

To a solution of 0.3 g of copper nitrate in 3 mol of ion exchanged water, was added a 25% aqueous ammonia solution to adjust pH to a range from 7.8 to 7.9, followed by addition of 1.0 g of a hydrotalcite [Mg₆Al₂(OH)₁₆CO₃] and stirring at room temperature for 0.5 hour. Next, 35 mL of ion exchanged water was further added, the mixture was stirred for 0.5 hour, the resulting solids were collected by filtration under reduced pressure, washed with deionized water, dried under reduced pressure, fired at 110°C for 12 hours, reduced at 180°C in a hydrogen atmosphere for 0.5 hour, and thereby yielded a catalyst including Cu particles immobilized on a hydrotalcite surface (Cu/HT).

### (EXAMPLE 3-1)

A mixture of 1 mmol of cyclooctanol, 5 mL of toluene, and 0.1 g (Cu: 3.0 percent by mole) of the catalyst prepared from Example 3-0 and including Cu particles immobilized on a hydrotalcite surface was stirred at 110°C in an argon atmosphere for 6 hours and thereby yielded a corresponding carbonyl compound (cyclooctanone) in a yield equivalent to a gas chromatographic (GC) yield of 99%.

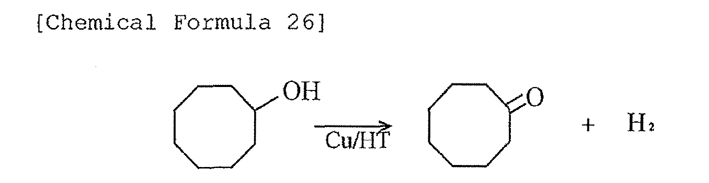

### (EXAMPLE 3-2)

The procedure of Example 3-1 was performed, except for carrying out a reaction in an oxygen atmosphere, and thereby yielded a corresponding carbonyl compound (cyclooctanone) in a yield equivalent to a gas chromatographic (GC) yield of 6%.

### (EXAMPLE 3-3)

A mixture of 1 mmol of cyclohexanol, 5 mL of toluene, and 0.1 g (Cu: 3.0 percent by mole) of the catalyst prepared from Example 3-0 and including Cu particles immobilized on a hydrotalcite surface was stirred at 110°C in an argon atmosphere for 24 hours and thereby yielded a corresponding carbonyl compound (cyclohexanone) in a yield equivalent to a gas chromatographic (GC) yield of 90%.

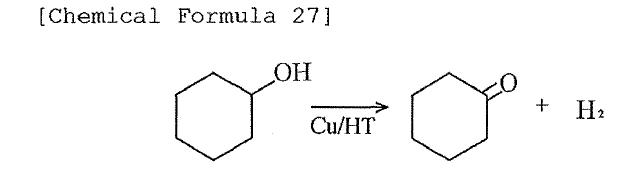

### (EXAMPLE 3-4)

The procedure of Example 3-3 was performed, except for carrying out a reaction in an oxygen atmosphere, and thereby yielded a corresponding carbonyl compound (cyclohexanone) in a yield equivalent to a gas chromatographic (GC) yield of 11%.

### (EXAMPLE 3-5)

A mixture of 1 mmol of 1-phenylethanol, 5 mL of toluene, and 0.1 g (Cu: 3.0 percent by mole) of the catalyst prepared from Example 3-0 and including Cu particles immobilized on a hydrotalcite surface was stirred at 110°C in an argon atmosphere for 9 hours and thereby yielded a corresponding carbonyl compound (acetophenone) in a yield equivalent to a gas chromatographic (GC) yield of 97%.

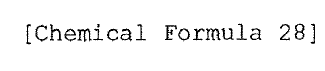

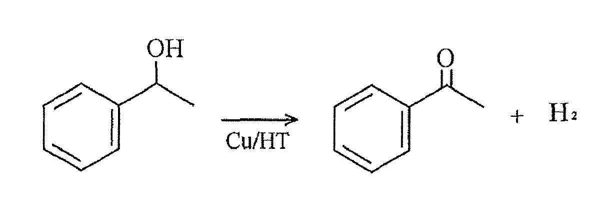

### (EXAMPLE 3-6)

The procedure of Example 3-5 was performed, except for carrying out a reaction in an oxygen atmosphere, and thereby yielded a corresponding carbonyl compound (acetophenone) in a yield equivalent to a gas chromatographic (GC) yield of 14%.

### (EXAMPLE 3-7)

A mixture of 1 mmol of benzyl alcohol, 5 mL of toluene, and 0.1 g (Cu: 3.0 percent by mole) of the catalyst prepared from Example 3-0 and including Cu particles immobilized on a hydrotalcite surface was stirred at 110°C in an argon atmosphere for 24 hours and thereby yielded a corresponding carbonyl compound (benzaldehyde) in a yield equivalent to a gas chromatographic (GC) yield of 19%.

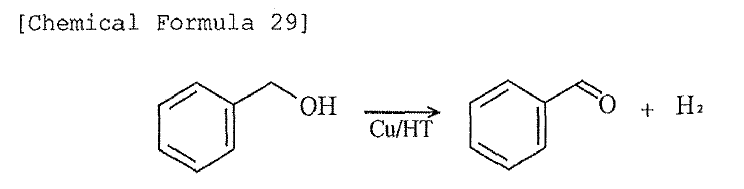

### (EXAMPLE 3-8)

The procedure of Example 3-7 was performed, except for carrying out a reaction in an oxygen atmosphere, and thereby yielded a corresponding carbonyl compound (benzaldehyde) in a yield equivalent to a gas chromatographic (GC) yield of 49%.

### (EXAMPLE 3-9)

A mixture of 1 mmol of 2-octanol, 5 mL of toluene, and 0.1 g (Cu: 3.0 percent by mole) of the catalyst prepared from Example 3-0 and including Cu particles immobilized on a hydrotalcite surface was stirred at 110°C in an argon atmosphere for 24 hours and thereby yielded a corresponding carbonyl compound (2-octanone) in a yield equivalent to a gas chromatographic (GC) yield of 94%.

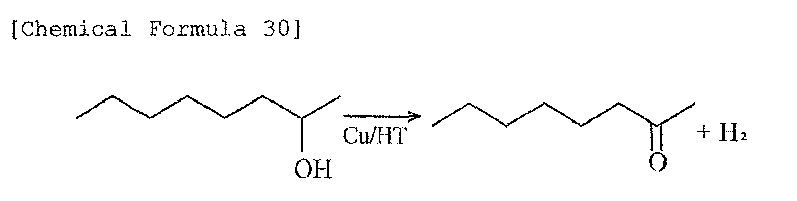

### (EXAMPLE 3-10)

The procedure of Example 3-9 was performed, except for carrying out a reaction in an oxygen atmosphere, and thereby yielded a corresponding carbonyl compound (2-octanone) in a yield equivalent to a gas chromatographic (GC) yield of 7%.

### (EXAMPLE 3-11)

A mixture of 1 mmol of n-octanol, 5 mL of toluene, and 0.1 g (Cu: 3.0 percent by mole) of the catalyst prepared from Example 3-0 and including Cu particles immobilized on a hydrotalcite surface was stirred at 110°C in an argon atmosphere for 24 hours and thereby yielded a corresponding carbonyl compound (n-octyl aldehyde; octanal) in a yield equivalent to a gas chromatographic (GC) yield of 2%.

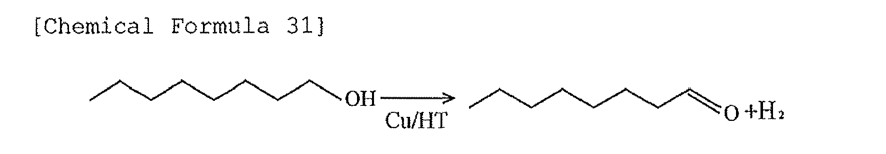

### (EXAMPLE 3-12)

The procedure of Example 3-11 was performed, except for carrying out a reaction in an oxygen atmosphere, and thereby yielded a corresponding carbonyl compound (n-octyl aldehyde) in a yield equivalent to a gas chromatographic (GC) yield of 4%.

### (EXAMPLE 3-13)

A mixture of 1 mmol of phenylcyclopropylmethanol, 5 mL of toluene, and 0.1 g (Cu: 3.0 percent by mole) of the catalyst prepared from Example 3-0 and including Cu particles immobilized on a hydrotalcite surface was stirred at 110°C in an argon atmosphere for 48 hours and thereby yielded a corresponding carbonyl compound (phenyl cyclopropyl ketone) in a yield equivalent to a gas chromatographic (GC) yield of 75%.

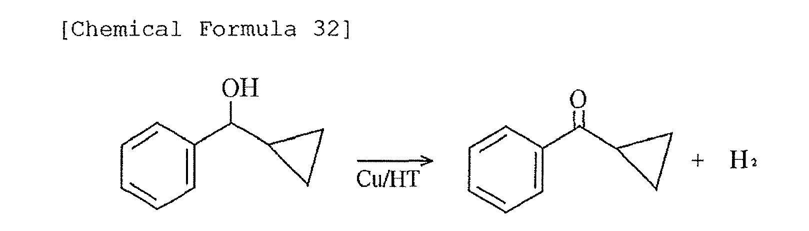

### (EXAMPLE 3-14)

A mixture of 1 mmol of 2-adamantanol, 5 mL of toluene, and 0.1 g (Cu: 3.0 percent by mole) of the catalyst prepared from Example 3-0 and including Cu particles immobilized on a hydrotalcite surface was stirred at 110°C in an argon atmosphere for 21 hours and thereby yielded a corresponding carbonyl compound (2-adamantanone) in a yield equivalent to a gas chromatographic (GC) yield of 89%.

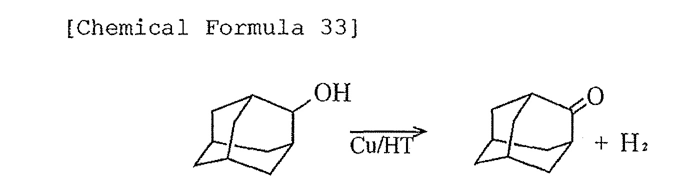

### (EXAMPLE 3-15)

A mixture of 1 mmol of 1-hydroxymethyladamantane, 5 mL of toluene, and 0.1 g (Cu: 3.0 percent by mole) of the catalyst prepared from Example 3-0 and including Cu particles immobilized on a hydrotalcite surface was stirred at 110°C in an argon atmosphere for 24 hours and thereby yielded a corresponding carbonyl compound (1-adamantanal) in a yield equivalent to a gas chromatographic (GC) yield of 6%.

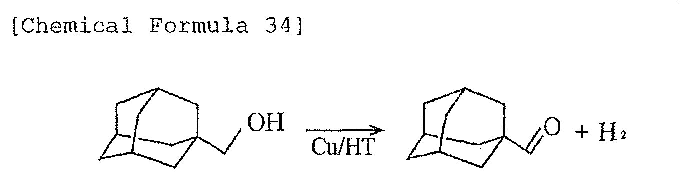

### (EXAMPLE 4)

A mixture of 1 mmol of 3-phenyl-2-propen-1-ol (cinnamyl alcohol), 5 mL of toluene, and 0.1 g of the catalyst prepared from Example 2-0 and including Ag particles immobilized on a hydrotalcite surface was stirred at 110°C in an argon atmosphere for 6 hours and thereby yielded a corresponding carbonyl compound (cinnamaldehyde), in a conversion from cinnamyl alcohol of 100% with a selectivity for cinnamaldehyde of 100%.

### (EXAMPLE 5)

### [Preparation of Catalyst Including Pd Particles Immobilized on Hydrotalcite Surface (Pd/HT)]

A hydrotalcite [Mg₆Al₂(OH)₁₆CO₃] was added to a solution of a Pd compound in ion exchanged water, followed by stirring. The resulting solids were collected by filtration under reduced pressure, washed with deionized water, dried under reduced pressure, fired, reduced in a hydrogen atmosphere, and thereby yielded a catalyst including Pd particles immobilized on a hydrotalcite surface (Pd/HT).

### [Production of Carbonyl Compound]

The procedure of Example 5 was performed, except for using the above-prepared catalyst including Pd immobilized on a hydrotalcite surface (Pd/HT) instead of the catalyst including Ag particles immobilized on a hydrotalcite surface. As a result, a conversion from cinnamyl alcohol was 100%, and a selectivity for cinnamaldehyde was 18%.

### (EXAMPLE 6)

### [Preparation of Catalyst Including Ru Particles Immobilized on Hydrotalcite Surface (Ru/HT)]

To a solution of RuCl₃·xH₂O in ion exchanged water, was added 1.0 g of a hydrotalcite [Mg₆Al₂(OH)₁₆CO₃], followed by stirring at room temperature. The resulting solids were collected by filtration under reduced pressure, washed with deionized water, dried under reduced pressure, fired, reduced in a hydrogen atmosphere, and thereby yielded a catalyst including Ru particles immobilized on a hydrotalcite surface (Ru/HT).

### [Production of Carbonyl Compound]

The procedure of Example 5 was performed, except for using the above-prepared catalyst including Ru particles immobilized on a hydrotalcite surface (Ru/HT) instead of the catalyst including Ag particles immobilized on a hydrotalcite surface. As a result, a conversion from cinnamyl alcohol was 99%, and a selectivity for cinnamaldehyde was 70%.

### Industrial Applicability

The present invention enables efficient dehydrogenation of alcohols by a simple operation to give corresponding carbonyl compounds in high yields. The methods according to the present invention are applicable to a wide variety of alcohols and, above all, enable efficient dehydrogenation of alicyclic alcohols by the catalysis of a solid catalyst in a liquid phase under mild conditions, in contrast to known techniques. The catalysts according to the present invention, which include metal particles immobilized on a hydrotalcite surface, can be prepared by a simple operation and are easily recoverable and reusable after the completion of reaction, and are extremely advantageous from the viewpoint of "green chemistry (sustainable chemistry)".

## Claims

1. A catalyst comprising a hydrotalcite and, immobilized on a surface thereof, particles of at least one metal selected from the group consisting of copper (Cu), silver (Ag), and gold (Au).

2. A method for producing a carbonyl compound, comprising the step of dehydrogenating an alcohol in the presence of a catalyst including a hydrotalcite and, immobilized on a surface thereof, particles of at least one metal selected from the group consisting of copper (Cu), silver (Ag), and gold (Au).

3. A method for producing a carbonyl compound, comprising the step of dehydrogenating an alcohol in the presence of a catalyst including a hydrotalcite and, immobilized on a surface thereof, particles of a metal, wherein dehydrogenation is carried out in the absence of oxygen.
